Europäisches Patentamt

European Patent Office

Office européen des brevets

(19)

(11) Publication number: **0 152 652**
**B1**

(12) **EUROPEAN PATENT SPECIFICATION**

(45) Date of publication of patent specification: **05.08.87**    (51) Int. Cl.⁴: **B 01 J 23/74, C 07 C 1/04**

(21) Application number: **84201919.2**

(22) Date of filing: **19.12.84**

(54) Catalyst activation.

(30) Priority: **31.01.84 NL 8400281**

(43) Date of publication of application:
**28.08.85 Bulletin 85/35**

(45) Publication of the grant of the patent:
**05.08.87 Bulletin 87/32**

(84) Designated Contracting States:
**AT BE DE FR IT NL SE**

(56) References cited:
**EP-A-0 104 672**
**EP-A-0 110 449**
**DE-A-3 122 157**

(73) Proprietor: SHELL INTERNATIONALE
RESEARCH MAATSCHAPPIJ B.V.
Carel van Bylandtlaan 30
NL-2596 HR Den Haag (NL)

(72) Inventor: **Minderhoud, Johannes Kornelis**
**Badhuisweg 3**
**NL-1031 CM Amsterdam (NL)**
Inventor: **Post, Martin Franciscus Maria**
**Badhuisweg 3**
**NL-1031 CM Amsterdam (NL)**

(74) Representative: Aalbers, Onno et al
P.O. Box 302
NL-2501 CH The Hague (NL)

Courier Press, Leamington Spa, England.

**Description**

The invention relates to a process for the activation of a catalyst which is to be used for the conversion of a mixture of carbon monoxide and hydrogen into hydrocarbons.

The preparation of hydrocarbons from a $H_2/CO$ mixture by contacting this mixture at elevated temperature and pressure with a catalyst is known in the literature as the Fischer-Tropsch hydrocarbon synthesis. Catalysts often used for the purpose comprise one or more metals from the iron group, together with one or more promotors, and a carrier material. These catalysts can suitably be prepared by the known techniques, such as precipitation, impregnation, kneading and melting. The products which can be prepared by using these catalysts usually have a very wide range of molecular weight distribution and, in addition to branched and unbranched paraffins, often contain considerable amounts of olefins and oxygen-containing organic compounds. Usually only a minor portion of the products obtained is made up of middle distillates. Of these middle distillates not only the yield but also the pour point is unsatisfactory. Therefore the direct conversion of $H_2/CO$ mixtures according to Fischer-Tropsch is not a very attractive route for the production of middle distillates on a technical scale.

In this patent application "middle distillates" should be taken to be hydrocarbon mixtures whose boiling range corresponds substantially with that of the kerosine and gas oil fractions obtained in the conventional atmospheric distillation of crude mineral oil. The middle distillation range lies substantially between about 150 and 360°C.

Recently a class of Fisher-Tropsch catalysts was found which have the property of yielding a product in which only very minor amounts of olefins and oxygen-containing compounds occur and which consists virtually completely of unbranched paraffins, a considerable portion of which paraffins boils above the middle distillate range. It has been found that the high-boiling part of this product can be converted in high yield into middle distillates by hydrocracking. As feed for the hydrocracking at least the part of the product is chosen whose initial boiling point lies above the final boiling point of the heaviest middle distillate desired as end product. The hydrocracking, which is characterised by a very low hydrogen consumption, leads to middle distillates with a considerably better pour point than those obtained in the direct conversion of a $H_2/CO$ mixture according to Fischer-Tropsch.

The Fischer-Tropsch catalysts belonging to the above-mentioned class contain silica, alumina or silica-alumina as carrier material and cobalt together with zirconum, titanium and/or chromium as catalytically active metals, in such quantities that the catalysts comprise 3—60 pbw of cobalt and 0.1—100 pbw of zirconium, titanium and/or chromium per 100 pbw of carrier material. The catalysts are prepared by depositing the metals involved on the carrier material by kneading and/or impregnation. For further information on the preparation of these catalysts by kneading and/or impregnation reference may be made to European patent application No. 127220.

Preparatory of being eligible for use in the preparation of hydrocarbons from a $H_2/CO$ mixture the cobalt catalysts should be activated. This activation may suitably be carried out by contacting the catalyst at a temperature between 200 and 350°C with hydrogen or a hydrogen-containing gas.

Further investigation into the activation and performance of the afore-mentioned cobalt catalysts has now shown that where stability is concerned, their performance is to a great extent dependent on the space velocity of the hydrogen-containing gas, the hydrogen partial pressure and overall pressure used during the activation as well as on the surface area, the cobalt load and the zirconium load of the catalyst to be activated.

It has been found that where their stability is concerned, the catalysts show optimum performance when the activation is carried out under such conditions as to satisfy the relation

$$\frac{D}{10^4 \times (P_{H_2})^2 \times P_{Tot}} > \frac{10 \times S}{L \times (Z+1)} \ ,$$

wherein

D = space velocity, expressed as $N1.1^{-1}.h^{-1}$,
$P_{H_2}$ = hydrogen partial pressure, as bar,
$P_{Tot}$ = overall pressure, as bar,
S = surface area of the catalyst, as $m^2/ml$,
L = cobalt load of the catalyst as mg Co/ml, and
Z = zirconium load of the catalyst as mg Zr/100 mg carrier.

The present patent application therefore relates to a process for the activation of a catalyst, in which a catalyst comprising 3—60 pbw of cobalt and 0.1—100 pbw of at least one other metal chosen from the group formed by zirconium, titanium and chromium per 100 pbw of silica, alumina or silica-alumina, which catalyst has been prepared by kneading and/or impregnation, is contacted at a temperature between 200 and 350°C with hydrogen or a hydrogen-containing gas under such conditions as to satisfy the afore-mentioned relation.

The process of the invention is preferably applied to the cobalt catalysts which form the subject matter of European patent application No. 127220. They are catalysts which satisfy the relation:

2

$$(3 + 4 R) > \frac{L}{S} > (0.3 + 0.4 R),$$

wherein

$L$ = the total quantity of cobalt present on the catalyst, expressed as mg Co/ml catalyst,

$S$ = the surface area of the catalyst, expressed as $m^2$/ml catalyst, and

$R$ = the weight ratio of the quantity of cobalt deposited on the catalyst by kneading to the total quantity of cobalt present on the catalyst.

Further the process of the invention is preferably applied to cobalt catalysts which have been prepared by one of the three procedures mentioned hereinafter:

a) first cobalt is deposited in one or more steps by impregnation and subsequently the other metal is deposited in one or more steps, also by impregnation.

b) first the other metal is deposited in one or more steps by impregnation and subsequently the cobalt is deposited in one or more steps, also by impregnation, and

c) first cobalt is deposited in one or more steps by kneading and subsequently the other metal is deposited in one or more steps by impregnation.

Further the process according to the invention is preferably applied to cobalt catalysts containing 15—50 pbw of cobalt per 100 pbw of carrier. The preferred quantity of other metal present in the cobalt catalysts depends on the way in which this metal has been deposited. In the case of catalysts where first cobalt has been deposited on the carrier, followed by the other metal, preference is given to catalysts containing 0.1—5 pbw of the other metal per 100 pbw of carrier. In the case of catalysts where first the other metal has been deposited on the carrier, followed by the cobalt, preference is given to catalysts containing 5—40 pbw of the other metal per 100 pbw of carrier. Preference is given to zirconium as the other metal and to silica as carrier material.

The catalysts activated according to the process of the invention are excellently suitable for use in the preparation of hydrocarbons from a mixture of carbon monoxide and hydrogen. The present patent application therefore also relates to a process for the preparation of hydrocarbons from a $H_2$-and-CO-containing feed by using a catalyst which has been activated according to the invention. The conversion of the $H_2$-and-CO-containing feed into hydrocarbons is preferably carried out at a temperature of 125—350°C and in particular of 175—275°C and a pressure of 5—100 bar and in particular of 10—75 bar.

$H_2$-and-CO-containing feeds which are eligible to be converted into hydrocarbons by using a catalyst activated according to the invention may very suitably be obtained from light hydrocarbons, such as natural gas, by steam reforming or partial oxidation. Suitable $H_2$-and-CO-containing feeds may also be obtained by separating a fraction containing unconverted hydrogen and carbon monoxide and other components, if desired, from the reaction product obtained by contacting a $H_2$/CO mixture with a catalyst containing one or more metal components having catalytic activity for the conversion of a $H_2$/CO mixture into hydrocarbons and/or oxygen-containing organic compounds.

The $H_2$-and-CO-containing feed which is converted into hydrocarbons by using a catalyst activated according to the invention preferably has a $H_2$/CO molar ratio higher than 1.5. If the feed has a $H_2$/CO molar ratio lower than 1.5, the latter is preferably raised to a value between 1.5 and 2.5 and in particular a value between 1.75 and 2.25 before it is contacted with the cobalt catalyst. The $H_2$/CO molar ratio of low-hydrogen feeds may be raised, for instance, by adding hydrogen, removing carbon monoxide, mixing with a hydrogen-rich $H_2$/CO mixture, or subjecting the low-hydrogen feed to the CO shift reaction.

The cobalt catalysts which have been activated according to the invention are very suitable for use in a process for the preparation of hydrocarbons containing at least five carbon atoms per molecule (hereinafter referred to as "$C_5^+$ hydrocarbons") from hydrocarbons containing at most four carbon atoms per molecule (hereinafter referred to as "$C_4^-$ hydrocarbons"), in which $C_4^-$ hydrocarbons are converted in the first step by steam reforming or partial oxidation into a $H_2$/CO mixture, which mixture is subsequently converted in a second step into a mixture of hydrocarbons substantially consisting of $C_5^+$ hydrocarbons by contacting it at elevated temperature and pressure with a cobalt catalyst activated according to the invention, in which the feed for the second step contains nitrogen and/or carbon dioxide as contaminants, which contaminannts are found in said feed substantially because the feed for the first step contained more than 20%v of nitrogen and/or carbon dioxide and/or because the first step was carried out by partial oxidation by using an oxygen-containing gas mixture containing more than 50%v of nitrogen.

Further the cobalt catalysts which have been activated according to the invention are very suitable for use in the preparation of $C_5^+$ hydrocarbons from $C_4^-$ hydrocarbons, in which $C_4^-$ hydrocarbons are converted in the first step by steam reforming into a $H_2$/CO mixture, which mixture is subsequently converted in a second step into a mixture of hydrocarbons consisting substantially of $C_5^+$ hydrocarbons by contacting it at elevated temperature and pressure with a cobalt catalyst activated according to the invention, in which the reaction product of the second step is divided into a gaseous fraction substantially consisting of unconverted hydrogen and carbon monoxide, $C_4^-$ hydrocarbons formed as by-product and carbon dioxide formed as by-product, and a liquid fraction substantially consisting of $C_5^+$ hydrocarbons and water at least part of which has been formed as by-product in the second step, in which the gaseous fraction is recycled to the first step and in which the excess hydrogen formed as well as the part of the steam added during the steam reforming which has remained unconverted are separated off during the process.

Finally the cobalt catalysts which have been activated according to the invention are very suitable for use in the preparation of $C_9^+$ hydrocarbons from $C_4^-$ hydrocarbons, in which $C_4^-$ hydrocarbons are converted in the first step by steam reforming into a $H_2/CO$ mixture, which mixture is subsequently converted in a second step into a mixture of hydrocarbons consisting substantially of $C_9^+$ hydrocarbons by contacting it at elevated temperature and pressure with a cobalt catalyst activated according to the invention, in which the reaction product of the second step is divided into a gaseous fraction consisting substantially of unconverted hydrogen and carbon monoxide, $C_8^-$ hydrocarbons formed as by-product and steam which has remained unconverted during the steam reforming as well as steam formed as by-product in the second step, and a liquid fraction consisting substantially of $C_9^+$ hydrocarbons, in which the gaseous fraction is recycled to the first step and in which the excess hydrogen formed in separated off during the process.

The three two-step processes described hereinbefore are preferably applied to a feed in which the $C_4^-$ hydrocarbons consist substantially of methane. Special preference is given to natural gas as feed.

As already observed hereinbefore, the present cobalt catalysts when used for the conversion of a $H_2$- and-CO-containing feed yield a substantially waxy product the high-boiling part of which can be converted in high yield into middle distillates by the use of hydrocracking. As feed for the hydrocracking at least the part of the product is chosen whose initial boiling point lies above the final boiling point of the heaviest middle distillate desired as end product. Although in the preparation of middle distillates from the product obtained over the cobalt catalyst the part of the product whose initial boiling point lies above the final boiling point of the heaviest middle distillate desired as end product will do as feed for the hydrocracking, it is preferred to use for this purpose the total $C_5^+$ fraction of the product prepared over the cobalt catalyst, since it has been found that the catalytic hydrotreatment leads to enhanced quality of the gasoline, kerosine and gas oil fractions present therein.

The hydrocracking is carried out by contacting the fraction to be treated at elevated temperature and pressure and in the presence of hydrogen with a catalyst comprising one or more noble metals from Group VIII supported on a carrier. The hydrocracking catalyst used by preference is a catalyst containing 0.1—2%w and in particular 0.2—1%w of one or more noble metals from Group VIII supported on a carrier. Preference is given to catalysts comprising platinum or palladium as Group VIII noble metal and silica-alumina as carrier. The hydrocracking is preferably carried out at a temperature of 200—400°C and in particular of 250—350°C and a pressure of 5—100 bar and in particular of 10—75 bar.

If the hydrocarbon synthesis carried out over the cobalt catalyst activated according to the invention is followed by a hydrocracking treatment for the preparation of middle distillates, the two steps can be carried out in 'series-flow', since the reaction product prepared over the cobalt catalyst will contain sufficient unconverted hydrogen for carrying out the hydrocracking. It is a matter of common knowledge that carrying out a multi-step process in 'series-flow' comprises using the total reaction product — without any components being removed therefrom or added thereto — of a certain step as a feed for the following step, which is carried out substantially at the same pressure as the preceding step.

The invention is now illustrated with the aid of the following example.

## Example
### Catalyst preparation

Eight $Co/Zr/SiO_2$ catalysts (Catalysts 1—8) were prepared by impregnation and/or kneading of silica carriers using solutions of cobalt and zirconium compounds. In each impregnation step the quantity of solution used had a volume which corresponded substantially with the pore volume of the carrier concerned. After each impregnation step the solvent was removed by heating and the material was calcined at 500°C. When a kneading step was used, the quantity of solution used has a volume which corresponded substantially with 150% of the pore volume of the carrier concerned. When a kneading step was used, the mixture was kneaded for three hours in a kneading machine. During the kneading a small portion of the solvent was removed by heating. After the kneading step the paste obtained was removed from the kneading machine, the remainder of the solvent was removed by heating and the material was ground and calcined at 500°C.

Catalysts 1—8 were prepared as follows.

### Catalyst 1

Kneading of a silica carrier with a solution of cobalt nitrate in water, followed by single-step impregnation of the cobalt-loaded carrier with a solution of zirconyl chloride in water.

### Catalyst 2

Single-step impregnation of a silica carrier with a solution of cobalt nitrate, followed by single-step impregnation of the cobalt-loaded carrier with a solution of zirconium nitrate in water.

### Catalyst 3

Single-step co-impregnation of a silica carrier with a solution of cobalt nitrate and zirconyl chloride in water.

### Catalysts 4—8

Three-step impregnation of a silica carrier with a solution of zirconium tetra n-propoxide in a mixture of n-propanol and benzene, followed by single-step impregnation of the zirconium-loaded carrier with a solution of cobalt nitrate in water.

### Catalysts 5—7

Two-step impregnation of a silica carrier with a solution of zirconium tetra n-propoxide in a mixture of n-propanol and benzene, followed by single-step impregnation of the zirconium-loaded carrier with a solution of cobalt nitrate in water.

Further information on Catalysts 1—8 is given in Table I.

### Catalyst activation

19 Activation experiments (1—19) were carried out in which treatment with hydrogen or a mixture of hydrogen and nitrogen was used to prepare starting from Catalysts 1—8, the activated Catalysts 1A—1F, 2A and 2B, 3A, 4A and 4B, 5A and 5B, 6A and 6B, 7A and 7B and 8A and 8B, respectively. The conditions used to carry out the activation experiments are given in Tables II and III.

### Catalyst trial

The activated catalysts 1A to 8B were used in nineteen experiments (I—XIX) in the preparation of hydrocarbons from a mixture of carbon monoxide and hydrogen of a $H_2/CO$ molar ratio of 2. The experiments were carried out in a reactor containing a fixed catalyst bed. The conditions used to carry out these experiments and the results of these experiments are given in Tables IV and V.

Of the activator experiments given in Tables II and III Experiments 3—6, 8, 11, 13—17 and 19 are experiments according to the invention. The catalysts obtained in these experiments, in which the relation which according to the invention should exist between catalyst properties and conditions used in the activation was satisfied, showed high stability, as is seen from the results given in Tables IV and V. Experiments 1, 2, 7, 9, 10, 12 and 18 fall outside the scope of the invention. They have been included in the patent application for comparison. The catalyst obtained in these experiments, in which the relation according to the invention between catalyst properties and conditions used during activation was not satisfied, showed low stability, as is seen from the results given in Tables IV and V.

TABLE I

| Catalyst No. | 1 | 2 | 3 | 4 | 5 | 6 | 7 | 8 |
|---|---|---|---|---|---|---|---|---|
| Co-load, pbw/100 pbw $SiO_2$ | 25 | 25 | 25 | 25 | 25 | 25 | 25 | 10 |
| Zr-load, pbw/100 pbw $SiO_2$ (Z) | 0.9 | 0.9 | 0.9 | 18 | 12 | 11 | 10 | 18 |
| L, mg Co/ml catalyst | 110 | 98 | 121 | 97 | 102 | 82 | 87 | 40 |
| S, $m^2$/ml catalyst | 25 | 96 | 17 | 100 | 100 | 102 | 100 | 30 |

TABLE II

| Catalyst to be activated No. | 1 | | | | | | 2 | | 3 |
|---|---|---|---|---|---|---|---|---|---|
| Activation experiment No. | 1 | 2 | 3 | 4 | 5 | 6 | 7 | 8 | 9 |
| Activated catalyst No. | 1A | 1B | 1C | 1D | 1E | 1F | 2A | 2B | 2C |
| Activation conditions | | | | | | | | | |
| $P_{Tot}$, bar | 1,1 | 2 | 1 | 1,3 | 1 | 1 | 1,3 | 2 | 1,1 |
| $P_{H_2}$, bar | 1,1 | 2 | 0,1 | 1 | 0,1 | 0,1 | 1 | 0,02 | 1,1 |
| T, °C | 250 | 250 | 250 | 250 | 240 | 280 | 250 | 250 | 250 |
| D, $Nl.l^{-1}.h^{-1}$ | 6000 | 6000 | 6000 | 48000 | 6000 | 6000 | 48000 | 1200 | 6000 |

0 152 652

TABLE III

| Catalyst to be activated No. | 4 | | 5 | | 6 | | 7 | | 8 | |
|---|---|---|---|---|---|---|---|---|---|---|
| Activation experiment No. | 10 | 11 | 12 | 13 | 14 | 15 | 16 | 17 | 18 | 19 |
| Activated catalyst No. | 4A | 4B | 5A | 5B | 6A | 6B | 7A | 7B | 8A | 8B |
| Activation conditions | | | | | | | | | | |
| $P_{Tot}$, bar | 1,1 | 1,3 | 1,8 | 1,3 | 1,3 | 2 | 2 | 2 | 1,1 | 1 |
| $P_{H_2}$, bar | 1,1 | 1 | 1,8 | 1 | 1 | 0,2 | 0,04 | 0,08 | 1,1 | 0,1 |
| T, °C | 250 | 250 | 250 | 250 | 250 | 250 | 260 | 260 | 250 | 250 |
| D, $Nl.l^{-1}.h^{-1}$ | 2000 | 180000 | 10000 | 90000 | 90000 | 12500 | 1200 | 1200 | 2000 | 6000 |

TABLE IV

| Experiment No. | I | II | III | IV | V | VI | VII | VIII | IX |
|---|---|---|---|---|---|---|---|---|---|
| Catalyst No. | 1A | 1B | 1C | 1D | 1E | 1F | 2A | 2B | 2C |
| <u>Conditions</u><br><br>temperature, °C | 220 | 220 | 220 | 220 | 230 | 230 | 220 | 220 | 220 |
| pressure, bar | 30 | 30 | 30 | 30 | 20 | 20 | 20 | 20 | 20 |
| space velocity,<br>$Nl.l^{-1}.h^{-1}$ | 800 | 800 | 600 | 600 | 900 | 900 | 1000 | 1000 | 600 |
| Initial CO,<br>conversion, %v | 72 | 69 | 85 | 87 | 83 | 82 | 85 | 84 | 78 |
| Loss of CO,<br>conversion,<br>%v/100 h | 4 | 10 | 2 | 2 | 2 | 2 | 9 | 2 | 21 |

TABLE V

| Experiment No. | X | XI | XII | XIII | XIV | XV | XVI | XVII | XVIII | XIX |
|---|---|---|---|---|---|---|---|---|---|---|
| Catalyst No. | 4A | 4B | 5A | 5B | 6A | 6B | 7A | 7B | 8A | 8B |
| Conditions | | | | | | | | | | |
| Temperature, °C | 220 | 220 | 220 | 220 | 220 | 220 | 220 | 220 | 220 | 220 |
| pressure, bar | 20 | 20 | 20 | 20 | 20 | 20 | 20 | 20 | 20 | 20 |
| space velocity, $Nl.l^{-1}.h^{-1}$ | 2000 | 2000 | 2000 | 2000 | 2000 | 2000 | 2000 | 2000 | 800 | 800 |
| Initial CO, conversion, %v | 75 | 74 | 60 | 72 | 69 | 61 | 60 | 67 | 72 | 70 |
| Loss of CO, conversion, % v/100 h | 7 | 3 | 15 | 3 | 3 | 2 | 2 | 2 | 7 | 3 |

# 0 152 652

## Claims

1. A process for the activation of a catalyst, characterized in that a catalyst comprising 3—60 pbw of cobalt and 0.1—100 pbw of at least one other metal chosen from the group formed by zirconium, titanium and chromium per 100 pbw of silica, alumina or silica-alumina, which catalyst has been prepared by kneading and/or impregnation, is contacted with hydrogen or a hydrogen-containing gas at a temperature in the range between 200 and 350°C under such conditions as to satisfy the relation:

$$\frac{D}{10^4 \times (P_{H_2})^2 \times P_{Tot}} > \frac{10 \times S}{L \times (Z+1)},$$

wherein
D = space velocity, as N1.1$^{-1}$.h$^{-1}$,
$P_{H_2}$ = hydrogen partial pressure, as bar,
$P_{Tot}$ = overall pressure, as bar,
S = surface area of the catalyst, as m$^2$/ml,
L = cobalt load of the catalyst as mg Co/ml, and
Z = zirconium load of the catalyst as mg Zr/100 mg carrier.

2. A process as claimed in claim 1, characterized in that the catalyst satisfies the relation:

$$(3 + 4\,R) > \frac{L}{S} > (0.3 + 0.4\,R),$$

wherein
L = the total quantity of cobalt present on the catalyst, expressed as mg Co/ml catalyst,
S = the surface area of the catalyst, expressed as m$^2$/ml catalyst, and
R = the weight ratio of the quantity of cobalt deposited on the catalyst by kneading to the total quantity of cobalt present on the catalyst.

3. A process as claimed in claim 1 or 2, characterized in that per 100 pbw of carrier the catalyst comprises 15—50 pbw of cobalt and either 0.1—5 pbw of the other metal if during its preparation cobalt was deposited first and the other metal next, or 5—40 pbw of the other metal if during its preparation the other metal was deposited first and cobalt next.

4. A process as claimed in any one of claims 1—3, characterized in that the catalyst comprises zirconium as other metal and silica as carrier.

5. A process for the preparation of hydrocarbons by catalytic reaction of carbon monoxide with hydrogen, characterized in that a H$_2$-and-CO-containing feed is contacted at a temperature of 125—350°C and a pressure of 5—100 bar with a catalyst activated according to the process claimed in any one of claims 1—4.

## Patentansprüche

1. Verfahren zur Aktivierung eines Katalysators, dadurch gekennzeichnet, daß ein 3—60 Gew.-Teile Kobalt und 0,1—100 Gew.-Teile wenigstens eines anderen Metalles, ausgewählt aus der aus Zirkon, Titan und Chrom gebildeten Gruppe, je 100 Gew.-Teile Siliziumdioxid, Aluminiumoxid oder Siliziumdioxid-Aluminiumoxid enthaltender Katalysator, der durch Kneten und/oder Imprägnieren bereitet worden ist, mit Wasserstoff oder einem Wasserstoff enthaltenden Gas bei einer Temperatur im Bereich zwischen 200 und 350°C unter solchen Bedingungen in Berührung gebracht wird, daß der nachfolgenden Beziehung genügt wird:

$$\frac{D}{10^4 \times (P_{H_2})^2 \times P_{Tot}} > \frac{10 \times S}{L \times (Z+1)},$$

worin:
D = Raumgeschwindigkeit, ausgedrückt als N1.1$^{-1}$.h$^{-1}$,
$P_{H_2}$ = Wasserstoffpartialdruck, ausgedrückt in bar,
$P_{Tot}$ = Gesamtdruck, ausgedrückt in bar,
S = Oberfläche des Katalysators, ausgedrückt in m$^2$/ml,
L = Kobaltbeladung des Katalysators, ausgedrückt als MgCo/ml, und
Z = Zirkon-Beladung des Katalysators, ausgedrückt in mg Zr/100 mg Träger.

11

# 0 152 652

2. Verfahren nach Anspruch 1, dadurch gekennzeichnet, daß der Katalysator der folgenden Beziehung entspricht:

$$(3 + 4\,R) > \frac{L}{S} > (0.3 + 0.4\,R),$$

worin:

L = Gesamtmenge des auf dem Katalysator vorliegenden Kobalts, ausgedrückt als mg Co/ml-Katalysator,

S = Oberfläche des Katalysators, ausgedrückt in m²/ml Katalysator, und

R = Gewichtsverhältnis der auf dem Katalysator durch Kneten abgelagerten Kobaltmenge zu der auf dem Katalysator vorliegenden gesamten Kobaltmenge.

3. Verfahren nach Anspruch 1 oder 2, dadurch gekennzeichnet, daß der Katalysator je 100 Gew.-Teile Träger 15 bis 50 Gew.-Teile Kobalt und entweder 0,1—5 Gew-Teile des anderen Metalles enthält, wenn während seiner Herstellung Kobalt zuerst und das andere Metall hierauf abgelagert wurden, oder 5—40 Gewichtsteile des anderen Metalles enthält, wenn während seiner Herstellung das andere Metall zuerst und hierauf Kobalt abgelagert wurden.

4. Verfahren nach einem der Ansprüche 1—3, dadurch gekennzeichnet, daß der Katalysator Zirkon als das andere Metall und Siliziumdioxid als den Träger umfaßt.

5. Verfahren zur Herstellung von Kohlenwasserstoffatomen durch katalytische Reaktion von Kohlenmonoxid mit Wasserstoff, dadurch gekennzeichnet, daß ein Wasserstoff und Kohlenmonoxid enthaltendes Einsatzmaterial bei einer Temperatur von 125—350°C und bei einem Druck von 5 bis 100 bar mit einem nach dem in einem der Ansprüche 1—4 beanspruchten Verfahren aktivierten Katalysator in Berührung gebracht wird.

## Revendications

1. Procédé d'activation d'un catalyseur, caractérisé en ce qu'on met en contact un catalyseur comprenant 3—60 parties en poids de cobalt et 0,1—100 parties en poids d'au moins un autre métal choisi dans le groupe formé par le zirconium, le titane et le chrome, pour 100 parties en poids de silice, d'alumine ou de silice-alumine, ce catalyseur ayant été préparé par malaxage et/ou imprégnation, avec de l'hydrogène ou un gaz contenant de l'hydrogène, à une température comprise entre 200 et 350°C, dans des conditions telles qu'elles satisfassent à la relation:

$$\frac{D}{10^4 \times (P_{H_2})^2 \times P_{Tot}} > \frac{10 \times S}{L \times (Z+1)},$$

dans laquelle

D = vitesse spatiale, exprimée en $N1.1^{-1}.h^{-1}$,

$P_{H_2}$ = pression partielle d'hydrogène, en bars,

$P_{Tot}$ = pression totale, en bars,

S = surface spécifique du catalyseur, en m²/ml,

L = charge de cobalt du catalyseur en mg de Co/ml, et

Z = charge de zirconium du catalyseur en mg de Zr/100 mg de support.

2. Procédé selon la revendication 1 caractérisé en ce que le catalyseur satisfait à la relation:

$$(3 + 4\,R) > \frac{L}{S} > (0,3 + 0,4\,R),$$

dans laquelle

L = quantité totale de cobalt présente sur le catalyseur, exprimée en mg de Co/ml de catalyseur,

S = surface spécifique du catalyseur, exprimée en m²/ml de cataalyseur, et

R = rapport pondéral de la quantité de cobalt déposée sur le catalyseur par malaxage à la quantité totale de cobalt présente sur le catalyseur.

3. Procédé selon la revendication 1 ou 2, caractérisé en ce que, pour 100 parties en poids du support, le catalyseur comprend 15—50 parties en poids de cobalt et soit 0,1—5 parties en poids de l'autre métal, si pendant sa préparation le cobalt a été déposé d'abord et l'autre métal ensuite, soit 5—40 parties en poids de l'autre métal, si pendant sa préparation l'autre métal a été déposé d'abord et le cobalt ensuite.

4. Procédé selon l'une quelconque des revendications 1 à 3, caractérisé en ce que le catalyseur comprend du zirconium comme autre métal et de la silice comme support.

5. Procédé pour la préparation d'hydrocarbures par réaction catalytique de monoxyde de carbone avec de l'hydrogène, caractérisé en ce qu'on met en contact une alimentation contenant $H_2$ et CO, à une température de 125—350°C et sous une pression de 5—100 bars, avec un catalyseur activé selon le procédé selon l'une quelconque des revendications 1 à 4.

12